# EUROPEAN PATENT SPECIFICATION

(11) **EP 3 661 477 B1**
(45) Date of publication and mention of the grant of the patent: **18.03.2026**
(21) Application number: 17761637.2
(22) Date of filing: 18.08.2017
(51) Int. Cl.: A61K 8/02, A61K 8/25, A61K 8/27, A61Q 11/00

(54) **CORE-SHELL SILICA AND METHOD FOR PRODUCING SAME**
KERN-SCHALE-SILICIUMDIOXID UND HERSTELLUNGSVERFAHREN DAFÜR
SILICE À NOYAU-ENVELOPPE ET SON PROCÉDÉ DE FABRICATION

(43) Date of publication of application: 10.06.2020
(73) Proprietor: Colgate-Palmolive Company, New York, NY 10022 (US)
(72) Inventor: TANG, Saide, Princeton, New Jersey 08540 (US); FEI, Lin, Kendall Park, New Jersey 08824 (US); CHOPRA, Suman, Monroe, New Jersey 08831 (US)
(74) Representative: Sonnenhauser, Thomas Martin
(86) International application number: PCT/US2017/047494
(87) International publication number: WO 2019/035839

(56) References cited:
- CN-A- 1 695 447
- US-A1- 2016 338 919
- ANONYMOUS: "ZINC NITRATE HEXAHYDRATE (NITRIC ACID, ZINC SALT)", 9 January 2018 (2018-01-09), XP055439237, Retrieved from the Internet <URL:http://www.chemicalland21.com/industrialchem/inorganic/ZINC NITRATE HEXAHYDRATE.htm> [retrieved on 20180109]

## Description

### BACKGROUND

Conventional oral care compositions often include one or more active and/or benefit agents to provide the oral care composition with additional desired properties. For example, conventional oral care compositions may include an antibacterial agent and a tartar-control agent to provide an antibacterial and an anti-tartar effect, respectively. While the inclusion of the active and/or benefit agents are capable of providing desired properties to the oral care compositions, the increasing number of active and/or benefit agents included in the oral care compositions may raise regulatory concerns, increase the possibility of aversion from the consumer (e.g., sensitivity, allergy, taste, etc.), and increase the likelihood of incompatibility with other components of the oral care compositions.

In view of the foregoing, oral care compositions may often incorporate core shell particles to transport, carry, or otherwise deliver active and/or benefit agents to surfaces of an oral cavity. For example, oral care composition may often utilize core shell silica (CSS) particles to deliver one or more active and/or benefit agents to the surfaces of the oral cavity. Particularly, the CSS particles have an anionic surface capable of delivering various metal ions (e.g., Zn²⁺, Ca²⁺, etc.) as active and/or benefit agents to the surfaces of the oral cavity. Conventional CSS particles, however, exhibit relatively low capacity and/or loading of the metal ions, thereby limiting the amount of the active and/or benefit agents delivered to the oral cavity.

What is needed, then, are improved core shell silica particles for oral care compositions and methods for making the core shell silica particles.

### BRIEF SUMMARY

This summary is intended merely to introduce a simplified summary of some aspects of one or more implementations of the present disclosure. Further areas of applicability of the present disclosure will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing a method as defined in the claims for preparing high loading core shell silica (CSS) particles, or CSS particles having a relatively greater amount of surface active silicate groups than CSS particles prepared according to conventional methods. The method of the invention comprises: contacting silica particles with a base comprising a first metal ion, which is a monovalent ion as defined in the claims, to produce core shell silica particles, each of the core shell silica particles comprising a silica core and a silicate of the first metal ion etched on a surface of the silica core; and contacting each of the core shell silica particles comprising the silicate of the first metal ion with an acidic aqueous solution comprising a metal salt having a second metal ion, wherein the second metal ion is a divalent ion, to produce core shell silica particles comprising the silica core and a silicate of the second metal ion on the surface of the silica core, wherein the acidic aqueous solution further comprises a humectant, wherein the humectant is at least one polyhydric alcohol.

As defined in the claims, the first metal ion is a monovalent ion, optionally a group 1 metal ion.

As defined in the claims, the humectant of the acidic aqueous solution is at least one polyhydric alcohol, such as glycerin, glycol, inositol, maltitol, mannitol, sorbitol, xylitol, propylene glycol, polypropylene glycol (PPG), and polyethylene glycol (PEG).

In at least one implementation, the humectant is sorbitol.

In at least one implementation, contacting each of the core shell silica particles with the acidic aqueous solution includes incrementally adding the core shell silica particles to the acidic aqueous solution.

In at least one implementation, the silica particles include at least one of precipitated silica, fumed silica, and fused silica.

In at least one implementation, the base includes at least one of sodium hydroxide, potassium hydroxide, sodium carbonate, potassium carbonate, trisodium phosphate, disodium phosphate, potassium phosphate, dipotassium phosphate, tetrasodium pyrophosphate, and tetrapotassium pyrophosphate.

In at least one implementation, the base includes potassium hydroxide or sodium hydroxide.

In at least one implementation, a weight ratio of the silica particles to the base is from 0.1:1 to less than 4:1, 0.1:1 to less than 3:1, 1.3:1 to 1.5:1, or 1.4:1.

As defined in the claims, the second metal ion is a divalent metal ion.

In at least one implementation, the metal salt is a zinc salt, and the second metal ion is Zn²⁺.

In at least one implementation, a weight ratio of the humectant to the base is from 4.5:1 to 8.5:1, 6.2:1 to 6.8:1, 6.3:1 to 6.5:1, or 6.4:1.

In at least one implementation, a weight ratio of the humectant to the metal salt is from 10:1 to 13:1, 11.4:1 to 11.6:1, or 11.5:1.

In at least one implementation, the acidic aqueous solution has a pH of less than 7, 3.5 to 4.5, or 4.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure.

### BRIEF DESCRIPTION OF THE DRAWINGS

The present disclosure is best understood from the following detailed description when read with the accompanying Figure. It is emphasized that, in accordance with the standard practice in the industry, various features are not drawn to scale. In fact, the dimensions of the various features may be arbitrarily increased or reduced for clarity of discussion.

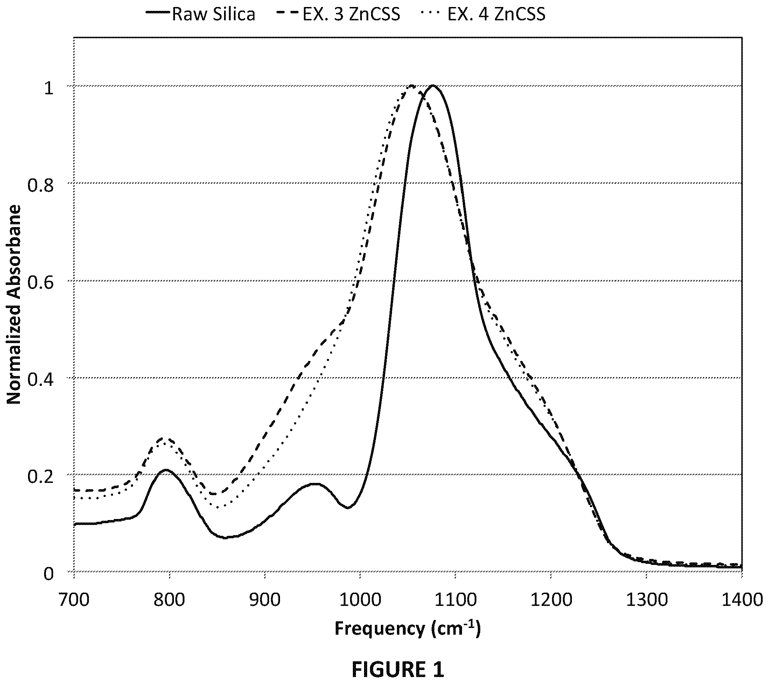

Figure 1 illustrates an FTIR spectrum of raw silica, and ZnCSS particles prepared according to Examples 3 and 4.

### DETAILED DESCRIPTION

The following description of various aspect(s) is merely exemplary in nature and is in no way intended to limit the disclosure, its application, or uses.

As used throughout, ranges are used as shorthand for describing each and every value that is within the range. Any value within the range may be selected as the terminus of the range.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

The present inventors have surprisingly and unexpectedly discovered methods for making or preparing high loading core shell silica (CSS) particles, or CSS particles having a relatively greater amount of surface active silicate groups than CSS particles prepared according to conventional methods.

### Methods

The present disclosure may provide methods for making or preparing core shell silica (CSS) particles having a relatively greater amount of surface active silicate groups than CSS particles prepared according to conventional methods. The method may include contacting silica (e.g., silica particles) and a base having a metal ion, such as a first metal ion, with one another to prepare CSS particles having a silica core and a surface etched with a silicate of the first metal ion, or first metal silicate. In at least one implementation, the silica may be admixed or otherwise dispersed in water, and the base, which may be provided as a solid or an aqueous solution, may be combined or otherwise contacted with the water to contact the silica with the base. In another implementation, the silica may be directly contacted with the base. For example, the silica may be contacted with an aqueous solution of the base. The method may also include reacting or otherwise contacting the CSS particles having their respective surfaces etched with the first metal silicate with a metal salt having a metal ion, such as a second metal ion, to provide the surface of the CSS particles with a silicate of the second metal ion, or second metal silicate. In a preferred implementation, the CSS particles having the first metal silicate may be contacted with an aqueous solution of the metal salt having the second metal ion. The aqueous solution of the metal salt having the second metal ion may have or be adjusted to have an acidic pH. The aqueous solution of the metal salt having the second metal ion includes a humectant.

As discussed above, the method may include contacting the silica (e.g., silica particles) with the base having the first metal ion to prepare the CSS particles having the silica core, where the surface of the silica core is etched with the silicate of the first metal ion, or the first metal silicate. As used herein, the term "etched," "etched silica," or "etched silica core," may refer to a silica core having a surface at least partially dissolved, where a metal silicate (e.g., the first metal silicate) is formed on, along, or about the silica core. It should be appreciated that the first metal silicates dispersed along the surface of the silica core are not additional layers coated on top of the original surface of the silica. For example, contacting the silica with the base may reduce respective diameters of the silica, thereby providing the silica core having the first metal silicate along the surface thereof. The first metal silicate formed along the surface of the silica core may be represented by formula (1),

M¹₂SiO₃ • xH₂O (1)

where M¹ is a monovalent metal ion, such as an alkali metal ion, and x is an integer from 0 to 10.

The base may be in the form of a solid or an aqueous solution. The base may have or include a first metal ion. The base may have a pKb of from about 0.1 to about 3. The first metal ion of the base is a monovalent metal ion as defined in the claims. In at least one implementation, the first metal ion of the base may be a group 1 or alkali metal ion. For example, the first metal ion of the base may be or include a sodium ion or a potassium ion. Illustrative bases may include, but are not limited to, sodium hydroxide, potassium hydroxide, sodium carbonate, potassium carbonate, trisodium phosphate, disodium phosphate, potassium phosphate, dipotassium phosphate, tetrasodium pyrophosphate, tetrapotassium pyrophosphate, and the like, and combinations thereof. In a preferred implementation, the base may be or include sodium hydroxide and/or potassium hydroxide. For example, the base may be a 50% aqueous solution of sodium hydroxide and/or a 45% aqueous solution of potassium hydroxide.

The silica may be or include any suitable silica capable of or configured to react with the base to form the CSS particles having the first metal silicate along the surface thereof. The silica may be in the form of particulates (e.g., silica particles), colloids (e.g., colloidal silica), amorphous silica, silica gel, and the like, and combinations thereof. Illustrative silica may be or include, but are not limited to, precipitated silica, fumed silica, fused silica, and the like, and combinations thereof. For example, the silica may be or include, but is not limited to, any one or more of ZEODENT^{®} 114, ZEODENT^{®} 105, ZEODENT^{®} 165, and the like, which are commercially available from Huber Engineered Materials of Atlanta, GA, SILODENT^{®} Silicas and SYLOID^{®} Silicas, which are commercially available from W.R. Grace of Houston, TX, SORBISIL^{®} Silicas, which are commercially available from PQ Corp. of Malvern, PA, AEROSIL^{®}, which is commercially available from Evonik Corp. of Parsippany, NJ, CAB-O-SIL^{®}, which is commercially available from Cabot Corp. of Alpharetta, GA, TECO-SIL^{®}, which is commercially available from C-E Minerals, Inc. of Roswell, GA, SPHERON^{®}, which is commercially available from Japanese Glass Co., TIXOSIL^{®} Silicas, which are commercially available from Solvay of Houston, TX, and the like, and combinations thereof. Illustrative silica may also be or include, but are not limited to, sodium silicates, which are discussed and described in U.S. Appl. No. 15/106,426, filed December 18, 2014, and published as U.S. Pub. No. 2016/0338919. The silica may also be or include, but are not limited to, silica abrasives, such as silica gels and precipitated amorphous silica.

The silica may have an average particle size of from 3 µm to 12 µm. For example, the silica, which may be colloidal particles/particulates, may have an average particle size of from 3 µm, 4 µm, 5 µm, 6 µm, or 7 µm to 8 µm, 9 µm, 10 µm, 11 µm, or 12 µm. In another example, the silica may have an average particle size of from 3 µm to 12 µm, 4 µm to 11 µm, 5 µm to 10 µm, 6 µm to 9 µm, or 7 µm to 8 µm.

A weight ratio of the amount of the silica to the amount of the base may vary widely. In at least one implementation, the weight ratio of the silica to the base may be from greater than or equal to 0.1:1 to less than 4:1. For example, the weight ratio of the silica to the base may be from 0.1:1, 0.5:1, 1.0:1, 1.5:1, 1.6:1, 1.7:1, 1.8:1, or 1.9:1 to 2.1:1, 2.2:1, 2.3:1, 2.4:1, 2.5:1, 3.0:1, 3.5:1, or less than 4.0:1. In another example, the weight ratio of the silica to the base may be from 0.1:1 to 4.0:1, 0.5:1 to 3.5:1, 1.0:1 to 3.0:1, 1.5:1 to 2.5:1, 1.6:1 to 2.4:1, 1.7:1 to 2.3:1, 1.8:1 to 2.2:1, or 1.9:1 to 2.1:1. In another implementation, the weight ratio of the silica to the base may be from greater than or equal to 0.1:1 to less than 3:1. For example, the weight ratio of the silica to the base may be from 0.1:1, 0.5:1, 1.0:1, 1.1:1, 1.2:1, 1.3:1, or 1.4:1 to 1.6:1, 1.7:1, 1.8:1, 1.9:1, 2.0:1, 2.5:1, or 3.0:1. In another example, the weight ratio of the silica to the base may be from 0.1:1 to 3.0:1, 0.5:1 to 2.5:1, 1.0:1 to 2.0:1, 1.1:1 to 1.9:1, 1.2:1 to 1.8:1, 1.3:1 to 1.7:1, or 1.4:1 to 1.6:1. In another implementation, the weight ratio of the silica to the base may be from 0.1:5, 0.2:5, 0.3:5, 0.5:5, 1:5, 1.5:5, or greater. In a preferred implementation, the weight ratio of the silica to the base may be from 1.3:1 to 1.7:1, 1.3:1 to 1.6:1, 1.3:1 to 1.5:1, or 1.4:1.

The CSS particles formed from contacting the silica particles with the base having the first metal ion, or the CSS particles including the first metal silicate, may have a relatively high charge density and/or ion exchange capacity. In at least one implementation, the CSS particles including the first metal silicate along the surface thereof may have a surface charge density of from about 0.5 meg/g of silica to about 4.5 meg/g of silica. For example, the CSS particles including the first metal silicate along the surface thereof may have a surface charge density of from about 0.5 meg/g of silica, about 1.0 meg/g of silica, about 1.5 meg/g of silica, or about 2.0 meg/g of silica to about 3.0 meg/g of silica, about 3.5 meg/g of silica, about 4.0 meg/g of silica, or about 4.5 meg/g of silica. In another example, the CSS particles including the first metal silicate along the surface thereof may have a surface charge density of from about 0.5 meg/g of silica to about 4.5 meg/g of silica, about 1.0 meg/g of silica to about 4.0 meg/g of silica, about 1.5 meg/g of silica to about 3.5 meg/g of silica, about 2.0 meg/g of silica to about 3.0 meg/g of silica, or from about 2.45 meg/g silica to about 2.55 meg/g silica.

The CSS particles formed from contacting the silica particles with the base having the first metal ion, or the CSS particles including the first metal silicate, may have a charge or ion exchange capacity of from about 0.05 C/cm² surface area to about 0.1 C/cm² surface area. For example, the CSS particles including the first metal silicate may have a charge or ion exchange capacity of from about 0.06 C/cm² surface area to about 0.1 C/cm² surface area, about 0.085 C/cm² surface area to about 0.095 C/cm² surface area, or about 0.089 C/cm² surface area.

As discussed above, the method may also include reacting or otherwise contacting the CSS particles having the first metal silicate along the surface thereof with a metal salt including the second metal ion to provide the surface of the CSS particles with a silicate of the second metal ion, or the second metal silicate. It should be appreciated that the second metal ion of the metal salt may displace or ion exchange with the monovalent metal ion of the first metal silicate disposed along the surface of the CSS particles. The second metal silicate formed along the surface of the silica core may be represented by formula (2),

M²SiO₃ • xH₂O (2)

where M² is a divalent metal ion, and x is an integer from 0 to 10. In a preferred embodiment, the second metal silicate formed along the surface of the silica core may be represented by formula (3),

Zn²SiO₃ • xH₂O (3)

where x is an integer from 0 to 10.

The metal salt may include any second metal ion capable of or configured to displace the monovalent metal ion of the first metal silicate. The second metal ion may be a group 2 or alkaline earth metal ion, a transition metal ion, a group 13 metal ion, a group 14 metal ion, and mixtures thereof. The second metal ion is a divalent metal ion as defined in the claims, other methods can use a trivalent metal ion, a tetravalent metal ion, and mixtures thereof. The second metal ion is a divalent metal ion as defined in the claims. Illustrative second metal ions may be or include, but are not limited to, Ca²⁺, Mg²⁺, Zn²⁺, Sn²⁺, Sr²⁺, Al³⁻, Zr⁴⁺, Ti⁴⁺, Fe³⁺, Fe²⁺, Mo²⁺, Co²⁺, Ni²⁺, Mn²⁺, Cu²⁺, Pd²⁺, Mo²⁺, Ru²⁺, and mixtures thereof.

Illustrative metal salts may be or include, but are not limited to, metal acetates, metal borates, metal butyrates, metal carbonates, metal halides, metal citrates, metal formates, metal gluconates, metal glycerates, metal glycolates, metal lactates, metal oxides, metal phosphates, metal picolinates, metal proprionates, metal salicylates, metal silicates, metal stearates, metal tartrates, metal undecylenates, and the like, and mixtures or combinations thereof. In a preferred embodiment the metal salt is a metal halide, such as a metal chloride. Illustrative metal chlorides may be or include, but are not limited to ZnCl₂, SnCl₂, SrCl₂, AlCl₃, FeCl₃, TiCl₄, ZrCl₄, and the like, and combinations thereof. In a preferred implementation, the metal salt is a zinc salt. Illustrative zinc salts may be or include, but are not limited to, zinc acetate, zinc borate, zinc butyrate, zinc carbonate, zinc chloride, zinc citrate, zinc formate, zinc gluconate, zinc glycerate, zinc glycolate, zinc lactate, zinc oxide, zinc phosphate, zinc picolinate, zinc proprionate, zinc salicylate, zinc silicate, zinc stearate, zinc tartrate, zinc undecylenate, and the like, and mixtures or combinations thereof. In a preferred implementation, the metal salt is zinc chloride (ZnCl₂).

The metal salt may be provided as a solid or an aqueous solution. For example, the metal salt may be provided as crystalline solids or powders. In another example, the metal salt may be dissolved or dispersed in an aqueous (e.g., water) or non-aqueous solution.

The method includes mixing or otherwise contacting the metal salt with a humectant prior to contacting the metal salt with the CSS particles having the first metal silicates. That is, the method includes preparing an aqueous solution comprising the metal salt and the humectant, and subsequently contacting the CSS particles having the first metal silicate with said aqueous solution of the metal salt and the humectant, or the metal salt/humectant solution. The humectant as defined in the claims is at least one polyhydric alcohol. Illustrative humectants may be or include, but are not limited to, polyhydric alcohol, such as glycerin, glycol, inositol, maltitol, mannitol, sorbitol, xylitol, propylene glycol, polypropylene glycol (PPG), polyethylene glycol (PEG), and the like, and mixtures or combinations thereof. Illustrative humectants may also be or include, but are not limited to, saccharide, such as fructose, glucose, sucrose, and the like, and combinations or mixtures thereof.

A weight ratio of the humectant to the base may vary widely. In at least one implementation, the weight ratio of the humectant to the base may be from greater than or equal to 0.5:1 to less than or equal to 10:1. For example, the weight ratio of the humectant to the base may be from 0.5:1, 1:1, 1.5:1, 2:1, 2.5:1, 3:1, 3.5:1, 4:1, 4.5:1, 5:1, 5.5:1, 6.0:1, 6.2:1, or 6.4:1 to 6.6:1, 6.8:1, 7:1, 7.5:1, 8:1, 8.5:1, 9:1, 9.5:1, or 10:1. In another implementation, the weight ratio of the humectant to the base may be from greater than or equal to 4.5:1 to less than or equal to 8.5:1. For example, the weight ratio of the humectant to the base may be from 4.5:1, 5:1, 5.5:1, 6.0:1, 6.2:1, or 6.4:1 to 6.6:1, 6.8:1, 7:1, 7.5:1, 8:1, or 8.5:1. In another example, the weight ratio of the humectant to the base may be from 4.5:1 to 8.5:1, 5:1 to 8:1, 5.5:1 to 7.5:1, 6.0:1 to 7:1, 6.2:1 to 6.8:1, or 6.4:1 to 6.6:1. In a preferred implementation, the weight ratio of the humectant to the base may be from 6.2:1 to 6.8:1, 6.3:1 to 6.5:1, or 6.4:1.

A weight ratio of the humectant to the metal salt may vary widely. In at least one implementation, the weight ratio of the humectant to the metal salt may be greater than or equal to 2:1 and less than or equal to 15:1. For example, the weight ratio of the humectant to the metal salt may be from 2:1, 2.5:1, 3:1, 3.5:1, 4:1, 4.5:1, 5:1, 5.5:1, 6:1, 6.5:1, 7:1, or 7.5:1 to 8:1, 8.5:1, 9:1, 9.5:1, 10:1, 10.5:1, 11:1, 11.5:1, 12:1, 12.5:1, 13:1, 13.5:1, 14:1, 14.5:1, or 15:1. In another implementation, the weight ratio of the humectant to the metal salt may be greater than or equal to 10:1 and less than or equal to 13:1. For example, the weight ratio of the humectant to the metal salt may be from 10:1, 10.5:1, 11:1, 11.2:1, or 11.4:1 to 11.6:1, 11.8:1, 12:1, 12.5:1, or 13:1. In another example, the weight ratio of the humectant to the metal salt may be from 10:1 to 13:1, 10.5:1 to 12.5:1, 11:1 to 12:1, 11.2:1 to 11.8:1, or 11.4:1 to 11.6:1. In a preferred implementation, the weight ratio of the humectant to the metal salt may be from 11.4:1 to 11.6:1, more preferably 11.5:1.

The metal salt/humectant solution may have an acidic pH. For example, the metal salt/humectant solution may have a pH of from greater than or equal to 1 to less than 7. In another example, the pH of the metal salt/humectant solution may be from 1, 2, 3, or 4 to 5, 6, or less than 7. In yet another example, the pH of the metal salt/humectant solution may be from 1 to 7, 2 to 6, or 3 to 5. In a preferred implementation, the metal salt/humectant solution may have a pH of from 3.5 to 4.5, more preferably 4.

As further described herein, the dropwise, stepwise, or otherwise incremental addition of the metal salt to the CSS particles having the first metal silicate may result in competing reactions that produces metal oxides (e.g., ZnO) and reduces the yield of the CSS particles having the second metal silicate. As such, contacting the CSS particles having the first metal silicate with the metal salt may include dropwise, stepwise, or otherwise incremental addition of the CSS particles having the first metal silicate to the metal salt. For example, contacting the CSS particles having the first metal silicate with the metal salt may include incrementally adding the CSS particles having the first metal silicate to an aqueous solution of the metal salt (e.g., ZnCl₂ solution). The aqueous solution of the metal salt may be maintained in a first vessel or container and the CSS particles having the first metal silicate may be maintained in a second vessel or container, and the CSS particles in the second container may be slowly or incrementally added to the first container such that only a relatively small amount of the CSS particles are available to react with the metal salt in the aqueous solution. In another example, contacting the CSS particles having the first metal silicate with the metal salt may include incrementally adding the CSS particles having the first metal silicate to a metal salt/humectant solution (e.g., aqueous solution of the metal salt and the humectant). In a preferred embodiment, the CSS particles having the first metal silicate may be incrementally added to an aqueous solution of ZnCl₂ and sorbitol, where the aqueous solution may have a pH of from 3 to 5, 3.5 to 4.5, or 4.

The CSS particles having the first metal silicate may be incremental added to the metal salt over a predetermined period of time. For example, the CSS particles having the first metal silicate may be incremental added to the metal salt over a period of at least 1 min, at least 2 min, at least 5 min, at least 10 min, at least 20 min, at least 30 min, at least 1 hour, at least 2 hours, at least 3 hours, at least 4 hours, at least 5 hours, at least 10 hours, at least 15 hours, at least 20 hours, at least 24 hours, at least 2 days, at least 4 days, or more.

The CSS particles formed from contacting the CSS particles having the first metal silicate with the metal salt, or the CSS particles including the second metal silicate, may include a silica core where the surface of the silica core is etched with the second metal silicate. It should be appreciated that the second metal silicate may be a silicate of the group 2 or alkaline earth metal ion, a transition metal ion, a group 13 metal ion, a group 14 metal ion, and mixtures thereof.

In at least one implementation, contacting the CSS particles including the first metal silicate with the metal salt may form or provide the surface of the CSS particles with the first metal silicates and the second metal silicates. For example, the second metal ion of the metal salt may substitute or ion exchange entirely or with only a portion of the first metal ions of the first metal silicates (i.e., incomplete ion exchange). In at least one implementation, the CSS particles may have a ratio of the first metal silicates to the second metal silicates of from 1:1 to 1:4. For example, the CSS particles may have a ratio of the first metal silicates to the second metal silicates of from 1:1, 1:1.5, or 1:2 to 1:3, 1:3.5, or 1:4. In another example, the CSS particles may have a ratio of the first metal silicates to the second metal silicates of from 1:1 to 1:4, 1:1.5 to 1:3.5, or 1:2 to 1:3. In another implementation, the CSS particles may have a ratio of the first metal silicates to the second metal silicates of from 1:1 to 1:10. For example, the CSS particles may have a ratio of the first metal silicates to the second metal silicates of from 1:1, 1:1.5, 1:2, 1:2.5, 1:3, 1:3.5, 1:4, 1 :4.5, or 1:5 to 1:6, 1:6.5, 1:7, 1:7.5, 1:8, 1:8.5, 1:9, 1:9.5, or 1:10. In another example, the CSS particles may have a ratio of the first metal silicates to the second metal silicates of from 1:1 to 1:10, 1:1.5 to 1:9.5, 1:2 to 1:9, 1:2.5 to 1:8.5, 1:3 to 1:8, 1:3.5 to 1:7.5, 1:4 to 1:7, 1:4.5 to 1:6.5, or 1:5 to 1:6.

In another implementation, contacting the CSS particles including the first metal silicate with the metal salt may form or provide the surface of the CSS particles with the second metal silicates. For example, all or substantially all of the first metal ion of the first metal silicate may ion exchange with the second metal ion of the metal salt. For example, at least 85%, at least 90%, at least 95%, at least 98%, or at least 99% of the first metal ion of the first metal silicate may ion exchange with the second metal ion of the metal salt.

In yet another implementation, the second metal silicate may be present in an amount of at least 30 weight %, at least 40 weight %, at least 50 weight %, at least 60 weight %, at least 70 weight %, at least 80 weight %, at least 90 weight %, or at least 95 weight % of the total metal silicate (i.e., first metal silicate and second metal silicate) of the CSS particles.

The CSS particles including the second metal silicate may be capable of being disposed in a dentin tubule of a human. For example, at least a portion of the CSS particles including the second metal silicate may be sized to be disposed into the dentin tubule of a human or non-human subject.

The CSS particles including the second metal silicate may include from greater than 0 weight % to less than or equal to 40 weight % of the second metal ion (e.g., Zn), based on a total weight of the CSS particles. For example, the amount or concentration of the second metal ion in the CSS particles may be from greater than 0 weight %, about 5 weight %, about 10 weight %, about 15 weight %, or about 20 weight % to about 25 weight %, about 30 weight %, or about 40 weight %, based on a total weight of the CSS particles. In another example, the amount or concentration of the second metal ion in the CSS particles may be from about 0 weight % to about 40 weight %, about 5 weight % to about 35 weight %, about 10 weight % to about 30 weight %, about 15 weight % to about 25 weight %, or about 20 weight % to about 25 weight %, based on a total weight of the CSS particles.

In at least one implementation, the amount of the second metal ion adsorbed to the surface of the CSS particles may be from greater than 0% to less than or equal to about 40% of a maximum ion-exchange capacity of the CSS particle for divalent ions. For example, the amount of the second metal ion adsorbed to the surface of the CSS particles may be from about 0%, about 5%, about 10%, about 15%, or about 20% to about 25%, about 30%, about 35%, or about 40%, of a maximum ion-exchange capacity of the CSS particle for divalent ions. In another example, the amount of the second metal ion adsorbed to the surface of the CSS particles may be from greater than 0% to about 40%, about 5% to about 35%, about 10% to about 30%, about 15% to about 25%, or about 20% to about 25% of a maximum ion-exchange capacity of the CSS particle for divalent ions.

### Compositions

The present disclosure may provide oral care compositions including the CSS particles disclosed herein. For example, the oral care compositions may include the core shell silica (CSS) particles as defined above and a carrier or orally acceptable vehicle. As used herein, "orally acceptable vehicle" may refer to a suitable vehicle, ingredient, or combination of ingredients, which can be used to form and/or apply the oral care composition to the surfaces of the oral cavity in a safe and effective manner. It should be appreciated that the orally acceptable vehicle may include materials such as, but not limited to, one or more antibacterial agents, anticalculus agents, buffers, additional abrasives, sources of peroxide (e.g., hydrogen peroxide), alkali metal bicarbonate salts, thickening materials, humectants, water, surfactants, titanium dioxide, cooling agents, coloring agents, and the like, and combinations thereof. For oral care compositions, the second metal ion is preferably zinc. It should be appreciated that zinc core shell silica (ZnCSS) may provide anti-bacterial and anti-malodor properties to the oral care compositions, and an improved taste profile over free zinc salts such as ZnCl₂.

In at least one implementation, the compositions (e.g., oral care compositions) may include from about 0.1 weight % to about 20 weight % of the CSS particles, based on a total weight of the composition. For example, the CSS particles may be present in an amount of about 0.1 weight %, about 2 weight %, about 4 weight %, about 6 weight %, about 8 weight %, or about 9 weight % to about 11 weight %, about 12 weight %, about 14 weight %, about 16 weight %, about 18 weight %, or about 20 weight %, based on a total weight of the composition. In another example, the CSS particles may be present in an amount of about 0.1 weight % to about 20 weight %, about 2 weight % to about 18 weight %, about 4 weight % to about 16 weight %, about 6 weight % to about 14 weight %, about 8 weight % to about 12 weight %, or about 9 weight % to about 11 weight %, based on a total weight of the composition.

The composition incorporating the CSS particles may be a solid, a paste, a gel, or a liquid. Illustrative compositions may be or include, but are not limited to, a dentifrice (e.g., a toothpaste, dental gel, dental cream, or tooth powder), a mouthwash, mouth rinse, or mouth spray, an oral slurry or liquid dentifrice, a gum or other confectionary, a lozenge, dental floss or dental tape, a prophylaxis paste or powder, a mono- or multi-layer oral film or gel strip (e.g., tooth strips or breath strips), functional film or gel flakes or functional micro- or nano-particles, a film-forming composition including pre-gel(s) or pre-polymer(s) (e.g., film-forming dentifrices), dental paints, a tooth hardener, or a coating on an oral device (e.g., orthodontic, appliance or implant).

### EXAMPLES

The examples and other implementations described herein are exemplary and not intended to be limiting in describing the full scope of compositions and methods of this disclosure. Equivalent changes, modifications and variations of specific implementations, materials, compositions and methods may be made within the scope of the present disclosure, with substantially similar results.

### Example 1

Core shell silica (CSS) particles having a first metal silicate were prepared. Particularly, silica (i.e., ZEODENT^{®} 105) was dispersed in hot water with a silica/water weight ratio of from about 0.3 to about 1.5. The temperature of this solution was from about 40 °C to about 70 °C. Then, a base (i.e., 45% KOH aqueous solution) was added into the silica slurry at a silica/base weight ratio of from about 0.2 to about 5. The reaction time was varied from 2 min to 20 min. The base etched surfaces of the silica to provide a silicate rich surface, thereby providing CSS particles having the first metal silicate along surfaces thereof. In this example, the CSS particles prepared had potassium silicates along the surface thereof, thereby providing potassium core shell silica (KCSS) particles.

### Example 2 (not claimed)

The KCSS particles prepared in Example 1 were utilized for the preparation of CSS particles having the second metal silicates. Particularly, zinc core shell silica (ZnCSS) particles were prepared according to a control or conventional method.

According to the control method, a metal salt, here a zinc chloride (ZnCl₂) powder, was added to the KCSS particles prepared in Example 1. Particularly, the ZnCl₂ powder was added to an aqueous dispersion of the KCSS prepared in Example 1, without heating. The mixing time was varied from 3 min to 20 min. It should be appreciated that the aqueous dispersion had a basic pH (about 11) due to the high concentration of KOH. Upon addition of the ZnCl₂, however, the solution solidified, thereby preventing further mixing.

Without being bound by theory, it is believed that the relatively high concentration of the base, or KOH, in the mixture resulted in the formation or precipitation of large amounts of metal hydroxides and/or metal oxides, such as zinc hydroxide (Zn(OH)₂) and/or zinc oxide (ZnO), respectively, via competing reactions. It is further believed that the zinc hydroxide and/or the zinc oxide that precipitated strongly interacted with one another to form networking or interacting structures that thickened or solidified the mixture. The solidification of the mixture prevented further reaction between the ZnCl₂ and the KCSS; and thus, prevented the formation or a high yield of the ZnCSS particles.

### Example 3

The KCSS particles prepared in Example 1 were utilized for the preparation of CSS particles having the second metal silicates. Particularly, zinc core shell silica (ZnCSS) particles were prepared according to a test or reverse addition method.

In the reverse addition method, the KCSS was added to a metal salt/humectant aqueous solution. Particularly, a humectant (i.e., sorbitol) in amounts varying from 15-35 g and ZnCl₂ in an amount of about 2 g was combined with water varying from 5 to 20 g to prepare the metal salt/humectant aqueous solution, and the KCSS particles, about 15-30 g, prepared from Example 1 were added into the metal salt/humectant aqueous solution. It was surprisingly and unexpectedly discovered that the addition of the KCSS particles of Example 1 to the metal salt/humectant aqueous solution resulted in a uniform opaque mixture/solution having the ZnCSS particles dispersed therein. It should be appreciated that the metal salt/humectant aqueous solution had an acidic pH of about 4.

Without being bound by theory, it is believe that the low, acidic pH conditions prevented the formation of the metal hydroxides and/or the metal oxides. Particularly, the acidic pH prevented the competing reactions that formed the Zn(OH)₂ and/or the ZnO, thereby allowing the substantially complete ion exchange between the ZnCl₂ and the potassium (K) of the KCSS.

### Example 4 (not claimed)

ZnCSS particles were prepared according to a traditional or conventional method, as discussed and described in U.S. Appl. No. 15/106,426, filed December 18, 2014, and published as U.S. Pub. No. 2016/0338919. Particularly, 2826.3 g of water and 224.6 g of a 45% KOH aqueous solution was combined with one another in a reaction chamber under mechanical stirring, and subsequently heated to a temperature above 100°C. The silica particles (ZEODENT^{®}105) in an amount of 560.1 g was incrementally or slowly added to the heated aqueous solution, and the mixture was heated/reacted for at least 4 hours to prepare the NaCSS colloids. Then, 180.18 g of ZnCl₂ was dissolved in 1 L of heated water (75°C) to prepare a ZnCl₂ solution. The ZnCl₂ solution was then slowly added to the KCSS colloidal suspension under stirring and allowed to react for at least 1 hour at 85°C. After adding the ZnCl₂ solution to the KCSS colloidal suspension, the mixture was allowed to cool to room temperature and stirred overnight. The resulting mixture, including the ZnCSS particles, was filtered to collect the ZnCSS particles, which were subsequently washed.

### Example 5

Analytical analysis was performed on raw silica particles (ZEODENT^{®}105), the ZnCSS particles prepared according to the methods Example 3, the reverse addition method, and Example 4, the conventional method. Particularly, Fourier transform infrared spectroscopy (FTIR) analysis was conducted on the raw silica (ZEODENT^{®}105) and the ZnCSS particles prepared according to Examples 3 and 4. The results of the FTIR are illustrated in Figure 1.

With continued reference to Figure 1, the peak at about 800 cm⁻¹ is associated with vibrations of the Si-O bond in silica, and the peak at about 960 cm⁻¹ is associated with the vibrations of the silicate group. As illustrated by the relatively high peak at 960 cm⁻¹ as compared to the raw silica particles (ZEODENT^{®}105), the ZnCSS particles prepared according to the reverse addition method of Example 3 exhibited a relatively greater amount of silicate groups than the ZnCSS prepared according to the conventional method of Example 4 and the raw silica particles. The results of the FTIR analysis demonstrated that the ZnCSS prepared by the reverse addition method of Example 3 exhibited a relatively greater amount of surface active silicate groups than the ZnCSS prepared by the conventional methods of Example 4.

### Example 6

Analytical analysis was performed on the silica (ZEODENT^{®}105) and the ZnCSS particles prepared according to the methods of Example 3, the reverse addition method, and Example 4, the conventional method. Particularly, electron spectroscopy for chemical analysis (ESCA) analysis was conducted on the silica (ZEODENT^{®}105) and the ZnCSS particles prepared according to Examples 3 and 4. ESCA was used to determine the surface composition of the respective ZnCSS particles. The results of the ESCA are summarized in Table 1.

**Table 1**

| **Summary of ESCA Analysis** | | | | | | | | |
|---|---|---|---|---|---|---|---|---|
| | **Atomic Percentage** | | | | | | **Atomic Ratio** | |
| **Sample** | **Oₜₒₜₐₗ** | **Si** | **O_{SiO3}** | **K** | **Zn** | **Cl** | **Si/O** | **O_{SiO3}/(2Zn+K)** |
| ZnCSS of Example 3 w/ 1.6% ZnCl₂ | 65.56 | 26.72 | 8.2 | 2.45 | 5.29 | 0 | 0.41 | 0.63 |
| ZnCSS of Example 3 w/ 2.0% ZnCl₂ | 66.54 | 25.92 | 5.46 | 1.05 | 6.50 | 0 | 0.39 | 0.39 |
| ZnCSS of Example 4 | 66.89 | 28.07 | 3.33 | 0.77 | 4.10 | 0.18 | 0.42 | 0.37 |
| Raw Silica ZEODENT^{®}105 | 69.30 | 30.30 | 0 | 0.41 | -- | -- | 0.44 | -- |

As illustrated in Table 1, the amount oxygen in silicate (O_{SiO3}) and Zinc in the ZnCSS particles prepared according to the reverse addition method of Example 3 was relatively higher (8.2%) than the O_{SiO3} of the ZnCSS particles prepared according to the conventional method of Example 4 (3.33%). The ESCA analysis demonstrated that the CSS prepared by the reverse addition method could provide CSS particles having as much as two times more surface silicate groups than CSS prepared according to the conventional method.

### Example 7

Calcium CSS particles (CaCSS) were prepared according to the reverse addition method and the conventional method. The procedures for the conventional method and the reverse addition method were similar to those disclosed in Example 3 and Example 4, respectively, except the ZnCl₂ was replaced with CaCl₂•2H₂O. ESCA analysis was conducted on the CaCSS prepared. The results of the ESCA are summarized in Table 2.

**Table 2**

| **Summary of ESCA Analysis** | | | | | | | | |
|---|---|---|---|---|---|---|---|---|
| | **Atomic Percentage** | | | | | | **Atomic Ratio** | |
| **Sample** | **Oₜₒₜₐₗ** | **Si** | **O_{SiO3}** | **K** | **Ca** | **Cl** | **Si/O** | **O_{SiO3}/(2Ca+K)** |
| CaCSS Reverse Addition | 68.23 | 27.42 | 7.65 | 0.28 | 4.07 | 0 | 0.40 | 0.91 |
| CaCSS Conventional | 67.72 | 28.25 | 4.85 | 1.43 | 2.13 | 0.48 | 0.42 | 0.85 |

As illustrated in Table 2, the CaCSS prepared according to the reverse addition method exhibited about 1.5 times more surface silicate groups and 2 times more surface calcium. The results demonstrate that the reverse addition method is not limited to zinc chloride, but may also be applied to other metal salts.

## Claims

1. A method, comprising:
contacting silica particles with a base comprising a first metal ion, wherein the first metal ion is a monovalent ion, to produce core shell silica particles, each of the core shell silica particles comprising a silica core and a silicate of the first metal ion etched on a surface of the silica core; and
contacting each of the core shell silica particles comprising the silicate of the first metal ion with an acidic aqueous solution comprising a metal salt having a second metal ion, wherein the second metal ion is a divalent ion, to produce core shell silica particles comprising the silica core and a silicate of the second metal ion on the surface of the silica core,
wherein the acidic aqueous solution further comprises a humectant, wherein the humectant is at least one polyhydric alcohol.

2. The method of claim 1, wherein the first metal ion is a group 1 metal ion.

3. The method of claim 1, wherein the humectant is at least one of glycerin, glycol, inositol, maltitol, mannitol, sorbitol, xylitol, propylene glycol, polypropylene glycol (PPG), and polyethylene glycol (PEG).

4. The method of claim 3, wherein the humectant is sorbitol.

5. The method of any one of claims 1 to 4, wherein contacting each of the core shell silica particles with the acidic aqueous solution comprises incrementally adding the core shell silica particles to the acidic aqueous solution.

6. The method of any one of claims 1 to 5, wherein the silica particles comprises at least one of precipitated silica, fumed silica, and fused silica.

7. The method of any one of claims 1 to 6, wherein the base comprises at least one of sodium hydroxide, potassium hydroxide, sodium carbonate, potassium carbonate, trisodium phosphate, disodium phosphate, potassium phosphate, dipotassium phosphate, tetrasodium pyrophosphate, and tetrapotassium pyrophosphate.

8. The method of any one of claims 1 to 7, wherein the base comprises potassium hydroxide or sodium hydroxide.

9. The method of any one of claims 1 to 8, wherein a weight ratio of the silica particles to the base is from 0.1:1 to less than 4:1, 0.1:1 to less than 3:1, 1.3:1 to 1.5:1, or 1.4:1.

10. The method of any one of claims 1 to 9, wherein the metal salt is a zinc salt, and the second metal ion is Zn²⁺.

11. The method of any one of claims 1 to 10, wherein a weight ratio of the humectant to the base is from 4.5:1 to 8.5:1, 6.2:1 to 6.8:1, 6.3:1 to 6.5:1, or 6.4:1.

12. The method of any one of claims 1 to 11, wherein a weight ratio of the humectant to the metal salt is from 10:1 to 13:1, 11.4:1 to 11.6:1, or 11.5:1.

13. The method of any one of claims 1 to 12, wherein the acidic aqueous solution has a pH of less than 7, 3.5 to 4.5, or 4.

## Patentansprüche

1. Verfahren, umfassend:
Inkontaktbringen von Siliziumdioxidpartikeln mit einer Base, die ein erstes Metallion umfasst, wobei das erste Metallion ein einwertiges Ion ist, um Kern-Schale-Siliziumdioxidpartikel herzustellen, wobei jedes der Kern-Schale-Siliziumdioxidpartikel einen Siliziumdioxidkern und ein Silikat des ersten Metallions umfasst, das auf eine Oberfläche des Siliziumdioxidkerns geätzt ist; und
Inkontaktbringen jedes der Kern-Schale-Siliziumdioxidpartikel, die das Silikat des ersten Metallions umfassen, mit einer sauren wässrigen Lösung, die ein Metallsalz mit einem zweiten Metallion umfasst, wobei das zweite Metallion ein zweiwertiges Ion ist, um Kern-Schale-Siliziumdioxidpartikel herzustellen, die den Siliziumdioxidkern und ein Silikat des zweiten Metallions auf der Oberfläche des Siliziumdioxidkerns umfassen,
wobei die saure wässrige Lösung weiterhin ein Feuchthaltemittel umfasst, wobei das Feuchthaltemittel mindestens ein mehrwertiger Alkohol ist.

2. Verfahren nach Anspruch 1, wobei das erste Metallion ein Metallion der Gruppe 1 ist.

3. Verfahren nach Anspruch 1, wobei das Feuchthaltemittel mindestens eines von Glycerin, Glykol, Inositol, Maltitol, Mannitol, Sorbitol, Xylitol, Propylenglykol, Polypropylenglykol (PPG) und Polyethylenglykol (PEG) ist.

4. Verfahren nach Anspruch 3, wobei das Feuchthaltemittel Sorbitol ist.

5. Verfahren nach einem beliebigen der Ansprüche 1 bis 4, wobei das Inkontaktbringen jedes der Kern-Schale-Siliziumdioxidpartikel mit der sauren wässrigen Lösung das schrittweise Hinzufügen der Kern-Schale-Siliziumdioxidpartikel zu der sauren wässrigen Lösung umfasst.

6. Verfahren nach einem beliebigen der Ansprüche 1 bis 5, wobei die Siliziumdioxidpartikel mindestens eines von gefälltem Siliciumdioxid, pyrogenem Siliciumdioxid und Quarzglas umfassen.

7. Verfahren nach einem beliebigen der Ansprüche 1 bis 6, wobei die Base mindestens eines von Natriumhydroxid, Kaliumhydroxid, Natriumcarbonat, Kaliumcarbonat, Trinatriumphosphat, Dinatriumphosphat, Kaliumphosphat, Dikaliumphosphat, Tetranatriumpyrophosphat und Tetrakaliumpyrophosphat umfasst.

8. Verfahren nach einem beliebigen der Ansprüche 1 bis 7, wobei die Base Kaliumhydroxid oder Natriumhydroxid umfasst.

9. Verfahren nach einem beliebigen der Ansprüche 1 bis 8, wobei ein Gewichtsverhältnis von Siliziumdioxidpartikel zu Base von 0,1:1 bis weniger als 4:1, 0,1:1 bis weniger als 3:1, 1,3:1 bis 1,5:1 oder 1,4:1 beträgt.

10. Verfahren nach einem beliebigen der Ansprüche 1 bis 9, wobei das Metallsalz ein Zinksalz ist und das zweite Metallion Zn²⁺ist.

11. Verfahren nach einem beliebigen der Ansprüche 1 bis 10, wobei ein Gewichtsverhältnis von Feuchthaltemittel zu Base von 4,5:1 bis 8,5:1, 6,2:1 bis 6,8:1, 6,3:1 bis 6,5:1 oder 6,4:1 beträgt.

12. Verfahren nach einem beliebigen der Ansprüche 1 bis 11, wobei ein Gewichtsverhältnis von Feuchthaltemittel zu Metallsalz von 10:1 bis 13:1, 11,4:1 bis 11,6:1 oder 11,5:1 beträgt.

13. Verfahren nach einem beliebigen der Ansprüche 1 bis 12, wobei die saure wässrige Lösung einen pH-Wert von weniger als 7, 3,5 bis 4,5 oder 4 aufweist.

## Revendications

1. Procédé, comprenant :
la mise en contact de particules de silice avec une base comprenant un premier ion métallique, dans lequel le premier ion métallique est un ion monovalent, pour produire des particules de silice noyau-enveloppe, chacune des particules de silice noyau-enveloppe comprenant un noyau de silice et un silicate du premier ion métallique gravé sur une surface du noyau de silice ; et
la mise en contact de chacune des particules de silice noyau-enveloppe comprenant le silicate du premier ion métallique avec une solution aqueuse acide comprenant un sel métallique ayant un second ion métallique, dans lequel le second ion métallique est un ion divalent, pour produire des particules de silice noyau-enveloppe comprenant le noyau de silice et un silicate du second ion métallique sur la surface du noyau de silice,
dans lequel la solution aqueuse acide comprend en outre un humectant, dans lequel l'humectant est au moins un alcool polyhydrique.

2. Procédé selon la revendication 1, dans lequel le premier ion métallique est un ion métallique du groupe 1.

3. Procédé selon la revendication 1, dans lequel l'humectant est au moins un humectant parmi la glycérine, le glycol, l'inositol, le maltitol, le mannitol, le sorbitol, le xylitol, le propylène glycol, le polypropylène glycol (PPG) et le polyéthylène glycol (PEG).

4. Procédé selon la revendication 3, dans lequel l'humectant est le sorbitol.

5. Procédé selon l'une quelconque des revendications 1 à 4, dans lequel la mise en contact de chacune des particules de silice noyau-enveloppe avec la solution aqueuse acide comprend l'ajout incrémentiel des particules de silice noyau-enveloppe à la solution aqueuse acide.

6. Procédé selon l'une quelconque des revendications 1 à 5, dans lequel les particules de silice comprennent au moins l'une de la silice précipitée, de la silice sublimée et de la silice fondue.

7. Procédé selon l'une quelconque des revendications 1 à 6, dans lequel la base comprend au moins l'un de l'hydroxyde de sodium, de l'hydroxyde de potassium, du carbonate de sodium, du carbonate de potassium, du phosphate trisodique, du phosphate disodique, du phosphate de potassium, du phosphate dipotassique, du pyrophosphate tétrasodique et du pyrophosphate tétrapotassique.

8. Procédé selon l'une quelconque des revendications 1 à 7, dans lequel la base comprend de l'hydroxyde de potassium ou de l'hydroxyde de sodium.

9. Procédé selon l'une quelconque des revendications 1 à 8, dans lequel un rapport pondéral des particules de silice à la base est de 0,1:1 à moins de 4:1, de 0,1:1 à moins de 3:1, de 1,3:1 à 1,5:1 ou de 1,4:1.

10. Procédé selon l'une quelconque des revendications 1 à 9, dans lequel le sel métallique est un sel de zinc, et le second ion métallique est Zn²⁺.

11. Procédé selon l'une quelconque des revendications 1 à 10, dans lequel un rapport pondéral de l'humectant à la base est de 4,5:1 à 8,5:1, de 6,2:1 à 6,8:1, de 6,3:1 à 6,5:1, ou de 6,4:1.

12. Procédé selon l'une quelconque des revendications 1 à 11, dans lequel un rapport pondéral de l'humectant au sel métallique est de 10:1 à 13:1, de 11,4:1 à 11,6:1 ou de 11,5:1.

13. Procédé selon l'une quelconque des revendications 1 à 12, dans lequel la solution aqueuse acide a un pH inférieur à 7, de 3,5 à 4,5 ou de 4.
